# EUROPEAN PATENT APPLICATION

(11) **EP 0 521 455 A2**
(43) Date of publication of application: **07.01.1993**
(21) Application number: 92111050.8
(22) Date of filing: 30.06.1992
(51) Int. Cl.: A61K 9/12, A61M 25/00, A61K 31/765

(54) **Aerosol composition containing a film-forming hydroxycarboxylic acid polymer**

(30) Priority: 02.07.1991 JP 161162/91
(71) Applicant: TAKEDA CHEMICAL INDUSTRIES, LTD., Chuo-ku, Osaka 541 (JP); Osaka Aerosol Industries Corporation, Osaka 550 (JP)
(72) Inventor: Yamada, Masayuki, Kawanishi, Hyogo 666-01 (JP); Mekata, Satoshi, Ibaraki, Osaka 567 (JP)
(74) Representative: von Kreisler, Alek, Dipl.-Chem.

(57) **Abstract**

The present invention relates to an aerosol composition which comprises a biodegradable hydroxycarboxylic acid polymer as a film-forming component.

This aerosol composition produces the following effects, among others: (1) application to the injured site by spraying will not cause pain; (2) it is biocompatible and resists peeling off after application; (3) it is not readily dissolved in or washed away with water; and (4) it can be allowed to remain on the skin after application since it is biodegradable.

## Description

### FIELD OF THE INVENTION

The present invention relates to an aerosol composition comprising a biodegradable hydroxycarboxylic acid polymer as a film-forming component.

### BACKGROUND OF THE INVENTION

Liquid disinfectant compositions have so far been used mainly in treating skin traumas, burns or the like. In cases where said traumas or burns are of a mild degree, the treatment comprises merely applying a liquid disinfectant composition to the affected areas. In cases where the degree is moderate or severe, it is common practice to disinfect the affected areas and then apply thereto an adhesive plaster or gauze so as to prevent or avoid pain on contact of the affected areas with something, delay in cure and, further, adhesion of dust, bacteria or the like on exposure to the open air and subsequent growth of bacteria or the like.

However, the problem is that the lesions, even when they are slight traumas, may sometimes suppurate as the result of invasion of dust, bacteria or the like. Covering the affected areas with an adhesive plaster or gauze may rather result in delayed cure due to reduced air passage and, further, detaching the adhesive plaster or gauze may result in damaging of the new granulation surface, leading to pain in the affected areas and/or delay in cure. Heretofore, liquid or aerosol compositions containing a cellulosic material or an acrylic resin have been developed as preparations for coating such affected areas (Japanese patent Laid Open Publication No. 290810/1990).

These compositions, when applied and allowed to dry, form films but, troublesomely, the films may peel off with the lapse of time or, when the film-forming component is a water-soluble substance, water may dissolve and wash away the films. In addition, the film-forming substances mentioned above are not biocompatible and may irritate the wounded skin; this presents another problem. Furthermore, when a nonaqueous solvent is used as the solvent, possible skin irritation by the solvent poses a further problem. Therefore, it is earnestly desired that a preparation improved in these respects be designed.

The object of this invention is to provide a film-forming substance which can form appropriately thick films on the injured or burnt skin or the like wounded surface and which is not soluble in water but is biocompatible and biodegradable. For dissolving such film-forming substance, a nonaqueous solvent is generally used. In that case, the nonaqueous solvent may act as a skin irritant in some instances and therefore it is necessary that a solvent which will not irritate the skin be selected in preparation designing. It is demanded that a preparation be designed which can cover the wounded surface or the like skin surface by forming a coat film having an appropriate thickness, namely a thickness sufficient to prevent dust or bacteria in the air from invading into the affected areas and, further, capable of ensuring sufficient oxygen permeation without preventing granulation. Said preparation should be free of stickiness and easily applicable. Aerosols may be mentioned as optimal preparation forms.

Under these circumstances, the present inventors made intensive investigations and, as a result, found that aerosol compositions containing, as a film-forming component, a biodegradable hydroxycarboxylic acid polymer, such as a lactic acid polymer (hereinafter referred to briefly as PLA), a glycolic acid polymer (hereinafter referred to briefly as PGA) or a lactic acid-glycolic acid copolymer (hereinafter referred to briefly as PLGA), can solve the above problems. Further investigations based on this finding have now led to completion of the present invention.

### SUMMARY OF THE INVENTION

The present invention is to provide an aerosol composition which comprises a biodegradable hydroxycarboxylic acid polymer as a film-forming component.

This aerosol composition produces the following effects, among other: (1) application to the injured site by spraying will not cause pain; (2) it is biocompatible and resists peeling off after application; (3) it is not readily dissolved in or washed away with water; and (4) it can be allowed to remain on the skin after application since it is biodegradable.

### DESCRIPTION OF THE PREFERRRED EMBODIMENT

The biodegradable hydroxycarboxylic acid polymer (hereinafter sometimes referred to merely as the polymer) to be incorporated, as a film-forming component, in the aerosol composition of this invention may be any homopolymer or copolymer of hydroxycarboxylic acid, said polymer being linked up by polyester bonding and capable of being degraded or decomposed by natural effectors after application to the damaged skin by spraying. The polymer preferably has a molecular weight of about 1,000 to 400,000, more preferably about 2,000 to 150,000.

Preferable example of the above-mentioned hydroxycarboxylic acid is a hydroxycarboxylic acid represented by the formula (I): 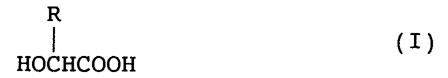
wherein R is hydrogen atom or a straight-chain or branched alkyl group having 1 to 8 carbon atoms. Examples of such hydroxycarboxylic acid include glycolic acid, lactic acid, 2-hydroxybutyric acid, 2-hydroxyvaleric acid, 2-hydroxy-3-methylbutyric acid, 2-hydroxycaproic acid, 2-hydroxyisocaproic acid, 2-hydroxycaprylic acid and so on. Particularly preferred is glycolic acid, lactic acid and 2-hydroxybutylic acid. These may each be D-, L- or D,L-configured.

The above-mentioned copolymer of hydroxycarboxylic acid may be a polymerization product of two or more different hydroxycarboxylic acids represented by the above-mentioned general formula (I). The mode of copolymerization of the copolymer may be random, block or graft.

As specific examples of the hydroxycarboxylic acid polymer, there may be mentioned PLA, PGA, PLGA, glycolic acid-2-hydroxybutylic acid copolymer and so on. These may be used either singly or in the form of a mixture of two or more of them.

The lactic acid/glycolic acid ratio (mole ratio) in PLGA is preferably 99/1 to 30/70, more preferably 99/1 to 40/60.

The glycolic acid/2-hydroxybutylic acid ratio (mole ratio) in the glycolic acid-2-hydroxybutylic acid copolymer is preferably 70/30 to 40/60.

The polymer used in the present invention can be produced by known methods. For example, it can be produced according to the method described in Japanese Patent Laid Open Publication No. 28521/1986 or No. 212436/1990, Japanese Patent Application No. 267929/1991 or USP 4,652,441.

The polymer is incorporated in the aerosol composition of this invention in an amount of 0.01% to 20% (weight by weight), preferably 0.02% to 10% (weight by weight), based on the whole amount of the aerosol composition of this invention.

In the aerosol composition of this invention, a propellant is generally used. As the propellant, there may be mentioned those used in conventional aerosol compositions, inclusive of dimethyl ether (hereinafter abbreviated as DME), liquefied propane gas (LPG), chlorofluorocarbons, N₂ gas, CO₂ gas, and substitutes for chlorofluorocarbons. When DME is used as the propellant, DME may serve also as a solvent, as mentioned below. It is also possible to use DME and some other propellant combinedly.

The amount of the propellant is not critical but, generally, it is 1% to 99% (weight by weight), preferably 40% to 90% (weight by weight), based on the whole amount of the aerosol composition of this invention.

Since the polymer mentioned above is insoluble in water, a solvent capable of dissolving the polymer may be used. DME is preferred as such solvent. Other good solvents for the polymer are ethanol, chloroform, acetone, and ethyl acetate, for instance. DME is not a skin irritant but is an aerosol propellant in wide use, hence it is preferred in the practice of this invention.

The amount of DME is not critical provided that it is sufficient to dissolve the polymer. Generally, DME is used in an amount of 1% to 99% (weight by weight) based on the whole aerosol composition of this invention.

In addition to the above-mentioned biodegradable hydroxycarboxylic acid polymer (film-forming component), solvent and propellant, a pharmacologically active ingredient, an insect repellent, an ulraviolet absorber, a nonbiodegradable film-forming component, a plasticizer, some other solvent than DME, a surfactant, a perfume and/or the like may further be incorporated in the aerosol composition of this invention. These additional components may suitably be selected from among known ones in conventional use.

More specifically, as the pharmacologically active ingredient, there may be mentioned skin disinfectants (e.g. isopropanol, ethanol, benzalkonium chloride, benzethonium chloride, iodine, potassium iodide, acrinol, chlorhexidine gluconate, salicylic acid, pyroxylin, hexachlorophene, boric acid, borax, sodium lauryl sulfate, thimerosal, methylrosaniline chloride, potassium permanganate, merbromin, urea, alkylpolyaminoethylglycine hydrochloride, etc.), pyostatic agents for external use (e.g. mafenide acetate, sulfadiazine, sulfisomidine sodium, sulfisoxazole, sulfamethoxazole sodium, sulfadiazine silver, erythromycin, oxytetracycline hydrochloride, tetracycline hydrochloride, tetracycline, chloramphenicol, fusidate sodium, mikamycin, fradiomycin sulfate, bacitracin, etc.), and analgesic, antipruritic, astringent or antiinflammatory agents (e.g. methyl salicylate, isothipendyl hydrochloride, diphenhydramine, diphenhydramine lauryl sulfate, zinc oxide, camphor, cantharis, capsicum tincture, ichthammol, lead acetate, turpentine oil, bismuth subgallate, fluocinolone acetonide, flumethasone pivalate, fluocinonide, fluorometholone, dexamethasone, betamethasone valerate, triamcinolone acetonide, prednisolone, hydrocortisone, diflucortolone valerate, ethyl aminobenzoate, indomethacin, glycyrrhetic acid, sodium dextransulfate, crotamiton, butyl fluphenamate, tannic acid, vitamin A, powdered gardenia fruit, youbaihi, etc.).

Other active ingredients that are generally used in pharmaceutical preparations for external use, such as dibucaine hydrochloride, naphazoline hydrochloride, chlorpheniramine maleate, l-menthol, peppermint oil, eucalyptus oil, epirocaine hydrochloride, procaine hydrochloride, dl-methylephedrine hydrochloride, allantoin, benzyl alcohol, nicotinic acid amide, tocopherol acetate, glycol salicylate, quaiazulene, lidocaine, d-borneol, glycerol, undecylenic acid, tolnaftate, clotrimazole and trichomycin, may be incorporated.

As the insect repellent, there may be mentioned, for example, pyrethroids.

As the ultraviolet absorber, there may be mentioned benzophenones, urocanic acid, paraaminobenzoic acid, Parasol A, and benzotriazoles, among others.

The level of addition of the pharmacologically active ingredient, insect repellent and/or ultraviolet absorber varies but, generally, it is within the range of about 0.001% to 30% (weight by weight) based on the whole aerosol composition of this invention.

The nonbiodegradable film-forming component may be of any kind provided that it assists the biodegradable hydroxycarboxylic acid polymer to form films. As specific examples, there may be mentioned acrylic resin. As the acrylic resin, there may be mentioned a homopolymer or copolymer of acrylic acid and ester thereof, acrylamide, acrylonitrile, methacrylic acid and ester thereof, and so on. As specific examples, there may be mentioned polyacrylic acid and sodium salt thereof and copolymer of (A) 2-ethylhexyl acrylate and (B) acrylic acid or ester thereof (e.g. methyl acrylate, ethyl acrylate, propyl acrylate, butyl acrylate, etc.), vinyl acetate or polyvinyl pyrrolidone. The acrylic resin preferably has a molecular weight of about 1,000 to 400,000. The component varies but is generally about 0.01% to 20% (weight by weight) based on the whole aerosol composition of this invention.

As the plasticizer, there may be mentioned vegetable oils, such surfactants as specifically mentioned below, and so forth. The addition level varies but is generally within the range of about 0.01% to 10% (weight by weight).

As other solvents than DME, there may be mentioned, for example, water, ethanol, isopropyl alcohol, n-butyl alcohol, propylene glycol, ethylene glycol, glycerol, methyl ethyl ketone, diethyl ether, isopropyl myristate, isopropyl palmitate, and castor oil. The level of addition thereof varies but, generally, it is about 0.01% to 50% (weight by weight) based on the whole aerosol composition of this invention.

As the surfactants, there may be mentioned, for example, Tween species and Span species. The level of addition thereof cannot be specified but is generally within the range of about 0.05% to 20% (weight by weight) based on the whole aerosol composition of this invention.

The perfume may be any of natural perfumes collected from plant sources or of synthetic perfumes such as alchols, aldehydes, ketones, esters and phenol ethers. The addition level thereof varies. Generally, however, the perfume is used in an amount of about 0.001% to 5% (weight by weight) based on the whole aersol composition of this invention.

The aerosol composition of this invention may further contain, in addition to the components mentioned above, one or more excipients which can be used in conventional aerosol compositions.

The container contents may be sprayed in mist form or spouted out in liquid form.

The aerosol composition of this invention can be produced in the conventional manner. More specifically, said composition can be produced without difficulty, for example by dissolving or dispersing the film-forming component, if desired together with one or more of the above-mentioned optional components, in the solvent and further admixing the propellant with the solution or dispersion. The aerosol composition prepared in such a manner is filled in pressure-resistant aerosol containers, whereupon it becomes ready for use.

The amount of the aerosol composition of this invention to be applied in each occasion varies but, generally, said composition should recommendably be used in an amount sufficient to wet or cover the skin portion damaged or affected by incised wound, excoriation, burn, athlete's foot or the like.

The duration of the effect of the aerosol composition of this invention after application may vary depending on the amount (dose) applied, the kind of the content and other factors. Generally, however, the effect will last for 6 hours to about 3 months after one application. The composition applied may be left as it stands since it spontaneously undergoes degradation thereafter. The frequency of application is not limited but, generally, up to four applications per day will be suitable. In case the affected areas are covered with an adhesive plaster or gauze, it becomes necessary to remove the covering. On the contrary, the aerosol composition of this invention need not be removed, so that it will not hurt the granules freshly formed on the affected surface.

### [Examples]

The following examples are further illustrative of the present invention but are by no means limitative of the scope of the invention.

### Example 1

An aerosol composition for skin disinfection and wound protection was prepared by filling a pressure-resistant aerosol container with 0.1 g of dibucaine hydrochloride, 0.1 g of naphazoline hydrochloride, 0.2 g of chlorpheniramine maleate and 0.1 g of benzethonium chloride, each as an active ingredient, 5 g of ethanol as a solvent, 2 g of PLA (Lyzomer™; Boehringer Ingelheim) as a film-forming component, and 92.5 g of DME as a solvent for PLA and as a propellant, and the container was fitted with an aerosol valve and push button. The aerosol composition thus obtained was applied to the skin by spraying and the skin was observed. Film formation (about 30 µm thick) was confirmed. The aerosol composition was further applied to an injured site. It could be administered without causing pain and the compatibility with the injured skin was good. Even when the thus-covered injured site came into contact with water, pain was not felt. Such phenomena as detachment of the film-forming substance were not observed, either.

### Example 2

An aerosol composition for skin disinfection and wound protection was prepared by filling a pressure-resistant aerosol container with 0.1 g of chlorhexidine gluconate and 0.9 g of lidocaine, each as an active ingredient, 5 g of water and 5 g of ethanol, each as a solvent, 1 g of PLA (Lyzomer™; Boehringer Ingelheim) and 0.5 g of PGA (Wako Pure Chemical Industries), each as a film-forming component, and 60 g of DME and 27.5 g of liquefied propane gas, each as a solvent for PLA and PGA and as a propellant, and the container was fitted with an aerosol valve and push button. The aerosol composition thus obtained was applied to the skin by spraying and the skin was observed. Film formation (about 30 µm thick) was confirmed. The aerosol composition was further applied to an injured site. It could be administered without causing pain and the compatibility with the injured skin was good. Detachment of the film-forming substance or the like phenomenon was not observed.

### Example 3

An aerosol composition for skin disinfection and wound protection was prepared by filling a pressure-resistant aerosol container with 0.1 g of dibucaine hydrochloride, 0.1 g of naphazoline hydrochloride, 0.2 g of chloropheniramine maleate and 0.1 g of benzethonium chloride, each as an active ingredient, 5 g of water as a solvent, 5 g of PLA (Biodegradable Polymer; Wako Pure Chemical Industries) as a film-forming component, and 89.5 g of DME as a solvent for PLA and as a propellant, and the container was then fitted with an aerosol valve and push button. The thus-obtained aerosol composition was applied to the skin by spraying and the skin was observed. Film formation (about 100 µm thick) was confirmed. The aerosol composition was further applied to an injured site. It could be administered without causing pain and the compatibility with the injured skin was good. Detachment of the film-forming substance or the like phenomenon was not observed.

### Example 4

An aerosol composition for skin disinfection and wound protection was prepared by filling a pressure-resistant aerosol container with 0.1 g of chlorhexidine gluconate and 0.9 g of lidocaine each as an active ingredient, 6 g of ethanol as a solvent, 3 g of PLA (Biodegradable Polymer; Wako Pure Chemical Industries) as a film-forming component, and 60 g of DME, 28.5 g of liquefied propane gas and 1.5 g of CO₂, each as a solvent for PLA and/or as a propellant, and the container was then fitted with an aerosol valve and push button. The thus-obtained aerosol composition was applied to the skin by spraying and the skin was observed. Film formation (about 50 µm thick) was confirmed. The aerosol composition was further applied to an injured site. It could be administerd without causing pain and the compatibility with the injured skin was good. Detachment of the film-forming substance or the like phenomenon was not observed.

### Example 5

An aerosol composition for athlete's foot treatment and wound protection was prepared by filling a pressure-resistant aerosol container with 1 g of clotrimazole, 5 g of crotamiton, 2 g of lidocaine and 1 g of l-menthol, each as an active ingredient, 20 g of ethanol as a solvent, 2 g of PLA (Biodegradable Polymer; Wako Pure Chemical Industries) as a film-forming component, 63 g of DME as solvent for PLA and as a propellant, and 5 g of isopropyl myristate and 1 g of monolaurin each as an excipient, and the container was then fitted with an aerosol valve and push button. The thus-obtained aerosol composition was applied to the skin by spraying and the skin was observed. Film formation (about 30 µm thick) was confirmed. The aerosol composition was further applied to an injured site. The compatibility with the injured skin was good. Detachment of the film-forming substance or the like phenomenon was not observed.

### Example 6

An aerosol composition for skin disinfection and wound protection was prepared by filling a pressure-resistant aerosol container with 0.1 g of dibucaine hydrochloride, 0.1 g of naphazoline hydrochloride, 0.2 g of chlorpheniramine maleate and 0.1 g of benzethonium chloride, each as an active ingredient, 24 g of ethanol and 6 g of water, each as a solvent, 1 g of PLA (Lactic acid polymer (PL3500DL); Taki Chemical Co., Ltd.) as a film-forming component, and 68.5 g of DME as a solvent for PLA and as a propellant, and the container was fitted with an aerosol valve and push button. The aerosol composition thus obtained was applied to the skin by spraying and the skin was observed. Film formation (about 15 µm thick) was confirmed. The aerosol composition was further applied to an injured site. It could be administered without causing pain and the compatibility with the injured skin was good. Even when the thus-covered injured site came into contact with water, pain was not felt. Such phenomena as detachment of the film-forming substance were not observed, either.

### Example 7

An aerosol composition for skin disinfection and wound protection was prepared by filling a pressure-resistant aerosol container with 0.1 g of dibucaine hydrochloride, 0.1 g of naphazoline hydrochloride, 0.2 g of chlorpheniramine maleate and 0.1 g of benzethonium chloride, each as an active ingredient, 14 g of ethanol as a solvent, 0.2 g of PLA (Lactic acid polymer (LA0005); Wako Pure Chemical Industries) as a film-forming component, and 79.3 g of DME as a solvent for PLA and as a propellant, and the container was fitted with an aerosol valve and push button. The aerosol composition thus obtained was applied to the skin by spraying and the skin was observed. Film formation (about 5 µm thick) was confirmed. The aerosol composition was further applied to an injured site. It could be administered without causing pain and the compatibility with the injured skin was good. Even when the thus-covered injured site came into contact with water, pain was not felt. Such phenomena as detachment of the film-forming substance were not observed, either.

### Reference Example

An aerosol composition for skin disinfection and wound protection was prepared by filling a pressure-resistant aerosol container with 0.1 g of dibucaine hydrochloride, 0.1 g of naphazoline hydrochloride, 0.2 g of chlorpheniramine maleate and 0.1 g of benzethonium chloride, each as an active ingredient, 5 g of ethanol as a solvent, 2 g of PLA (Lyzomer™; Boehringer Ingelheim) as a film-forming component, and 92.5 g of liquefied propane gas, and the container was then fitted with an aerosol valve and push button. Attempts were made to apply the thus-obtained aerosol composition to the skin by spraying. However, the valve was stopped up and smooth application could not be attained. After forced application by spraying, the skin site was observed. Film formation was not found.

## Claims

1. An aerosol composition which comprises a biodegradable hydroxycarboxylic acid polymer as a film-forming component.

2. An aerosol composition as claimed in Claim 1, wherein the biodegradable hydroxycarboxylic acid polymer is a homopolymer or copolymer of hydroxycarboxylic acid represented by the formula (I): 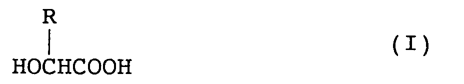 wherein R is hydrogen atom or a straight-chain or branched alkyl group having 1 to 8 carbon atoms, which is linked up by polyester bonding.

3. An aerosol composition as claimed in Claim 1, wherein the biodegradable hydroxycarboxylic acid polymer is a lactic acid polymer.

4. An aerosol composition as claimed in Claim 1, wherein the biodegradable hydroxycarboxylic acid polymer is a lactic acid-glycolic acid copolymer.

5. An aerosol composition as claimed in Claim 1, wherein the biodegradable hydroxycarboxylic acid polymer has a molecular weight of 1,000 to 400,000.

6. An aerosol composition as claimed in Claim 1, wherein the biodegradable hydroxycarboxylic acid polymer is incorporated in an amount of 0.01w/w% to 20w/w%, based on the whole amount of the aerosol composition.

7. An aerosol composition as claimed in Claim 1, wherein dimethyl ether is used as a propellant.

8. An aerosol composition as claimed in Claim 1 which further comprises a pharmacological active ingredient.
